# EUROPEAN PATENT APPLICATION

(11) **EP 3 869 516 A1**
(43) Date of publication of application: **25.08.2021**
(21) Application number: 21167851.1
(22) Date of filing: 07.05.2016
(51) Int. Cl.: G16H 20/17, G16H 20/10

(54) **BLOOD GLUCOSE MANAGEMENT SYSTEM**

(30) Priority: 08.05.2015 US 201562158953 P
(62) Divisional of application: 16723905.2
(71) Applicant: POPS! Diabetes Care, Inc., Stillwater MN 55082 (US)
(72) Inventor: CHRISTENSEN, Curtis, Stillwater, Minnesota 55082 (US); DAVIS, Daniel, Hugo, Minnesota 55038 (US); DAVIS, Erik, Forest Lake, Minnesota 55025 (US); STORMO, Chandler, Stillwater, Minnesota 55082 (US); STORMO, Lonny, Stillwater, Minnesota 55082 (US)
(74) Representative: Balsamo, Andrea

(57) **Abstract**

Methods and systems are disclosed for managing diabetes. Some embodiments can prompt a patient to take a blood glucose management action via a diabetes management application on the patient's mobile device. Some embodiments can collect context-related information concerning a patient's blood glucose measurement via the patient's mobile device. Some embodiments can enable categorization of a plurality of patients into diabetes risk categories for caregivers and others.

## Description

### RELATED APPLICATIONS

The present application claims priority to U.S. Provisional Application 62/158,953, filed May 8, 2015, the entire contents of which is incorporated herein by reference.

### TECHNICAL FIELD

This disclosure relates to tracking blood glucose levels and other information for the management of medical conditions.

### BACKGROUND

Managing a patient's blood glucose levels can be challenging. Generally, a patient must provide a blood sample to a meter multiple times each day and must self-assess based on the blood glucose measurements and associated trends, along with other factors he/she thinks may be influencing the measurements. The patient typically provides the blood sample by lancing his/her finger (or other body part) with a lancing device, applying the blood sample to a separate test strip, and introducing the test strip to the meter-three separate components are required. This arrangement often means patients are less likely to test their blood glucose levels as often as recommended and that any conclusions drawn from the measurement are based on incomplete information. Moreover, it can be difficult to provide relevant blood glucose information to others for whom accessing such information may be beneficial.

### SUMMARY

Embodiments described herein enable more effective management of diabetes. A diabetes management application running on a patient's mobile device can strategically prompt the patient to take one or more specific actions (e.g., take a blood glucose measurement, take in insulin and/or carbohydrates, exercise, etc.) to manage his/her blood glucose. The prompts can be triggered in the diabetes management application based on a variety of factors, such as information in the mobile device or accessible by the mobile device that bears on the patient's diabetes or management thereof.

In some embodiments, the diabetes management application receives a blood glucose measurement from the patient via an associated blood glucose monitor and collects context-related information corresponding to the blood glucose measurement. The context-related information can shed light on what the patient was doing in the time surrounding the blood glucose measurement. The context-related information can, in some cases, provide the historical backdrop for the patient and the blood glucose measurement. The diabetes management application can package the blood glucose measurement together with the context-related information for further investigation and analysis. In many embodiments, the diabetes management application can provide the packaged blood glucose measurement and context-related information to a remote server for storage and analysis by others.

In some embodiments, the remote server can receive diabetes information from a variety of patients in many different environments. In embodiments in which the server receives packages of blood glucose measurements and context-related information from patients, the server can categorize the patients into diabetes risk categories. In preferred embodiments, the diabetes risk categorization can be based on blood glucose measurements, the frequency with which patients provide blood glucose measurements, and various context-related information. A caregiver can instruct the server to display information associated with all the patients for whom he/she is providing care or only information associated with a selected subset of patients (e.g., only patients in a certain diabetes risk category). The server can also monitor trends, such as when a patient moves from one diabetes risk category to another or moves specified degrees within a diabetes risk category.

In many embodiments, patients can measure their blood glucose levels using a single piece of equipment: a blood glucose monitor coupled to a mobile electronic device. The blood glucose monitor can wirelessly communicate blood glucose measurements to an application on the mobile device, and the blood glucose measurements can be displayed to the patient on the mobile device. In many embodiments, the diabetes management application can perform a variety of analyses on the blood glucose measurements. The diabetes management application can ask the patient context-related questions based on one or more of the analyses it performs. In some embodiments, such questions can be posed to the patient in a manner that allows the patient to provide answers easily (e.g., via yes/no slider bars). In many instances, information from patients' answers to such context-related questions can lead to more robust conclusions concerning management of the patients' blood glucose levels. In some embodiments, the diabetes management application can provide incentives to the patient to encourage the patient to test their blood glucose levels as often as recommended. In some embodiments, the diabetes management application can provide educational information to the patient to help inform ongoing blood glucose management.

Embodiments described herein enable communication between the diabetes management application and a wider automated collaborative care system (ACC system). The ACC system can include a server that receives blood glucose measurements and other information from the diabetes management application and provides relevant feedback to the diabetes management application. In some embodiments, analyses of blood glucose information are performed on the diabetes management application, and analyzed information is provided to the ACC server. In some embodiments, the diabetes management application provides un-analyzed information to the ACC server, and the ACC server analyzes the information. In some embodiments, the diabetes management application and the ACC server share analytical responsibility. For example, the diabetes management application or the ACC server or both can determine which context-related questions to pose to a patient based on analyses of the patient's blood glucose measurement information.

Embodiments described herein enable patients to provide relevant information about their blood glucose levels to other interested parties. In some embodiments, patients can authorize healthcare providers and other caregivers (among others) to access their blood glucose information to enhance management of their blood glucose levels. In many instances, the other interested parties can access such information as part of the ACC system.

### BRIEF DESCRIPTION OF DRAWINGS

FIG. 1 is a schematic diagram of an illustrative ACC system.
FIG. 2 shows an illustrative blood glucose monitor and a mobile device.
FIG. 3 shows an illustrative blood glucose monitor and a mobile device.
FIG. 4 shows the blood glucose monitor and mobile device of FIG. 2, with the cover of the blood glucose monitor removed.
FIG. 5 is a flow chart depicting the processing of information related to patient prompts.
FIG. 6 is a flow chart depicting the processing of information related to context-related information.
FIG. 7 is an illustrative user interface of a diabetes management application in accordance with embodiments of the present invention.
FIG. 8 shows a patient using the blood glucose monitor of FIG. 1 by lancing his/her finger on an enclosed lancet.
FIG. 9 shows the patient of FIG. 8 applying a blood sample to a biological strip.
FIG. 10 is an illustrative user interface of a diabetes management application in accordance with embodiments of the present invention.
FIG. 11 is an illustrative user interface of a diabetes management application in accordance with embodiments of the present invention.
FIG. 12 is a flow chart depicting the processing of information related to a server.
FIG. 13 is an example of a set of risk categories.
FIG. 14 is an example of a display.
FIG. 15 is a schematic diagram of an illustrative ACC system in accordance with embodiments of the present invention.
FIG. 16 is a flow chart showing a portion of an illustrative ACC system in accordance with embodiments of the present invention.
FIG. 17 is a flow chart showing a portion of an illustrative ACC system in accordance with embodiments of the present invention.
FIG. 18 is an illustrative user interface of a diabetes management application in accordance with embodiments of the present invention.
FIG. 19 is an illustrative user interface of a diabetes management application in accordance with embodiments of the present invention.
FIG. 20 is an illustrative user interface of a diabetes management application in accordance with embodiments of the present invention.

### DETAILED DESCRIPTION

The following detailed description is exemplary in nature and is not intended to limit the scope, applicability, or configuration of the invention in any way. Rather, the following description provides practical illustrations for implementing exemplary embodiments of the present invention. Examples of constructions, materials, dimensions, and manufacturing processes may be provided for selected elements, and all other elements employ that which is known to those of skill in the field of the invention. Those skilled in the art will recognize that many of the examples provided have suitable alternatives that can be utilized.

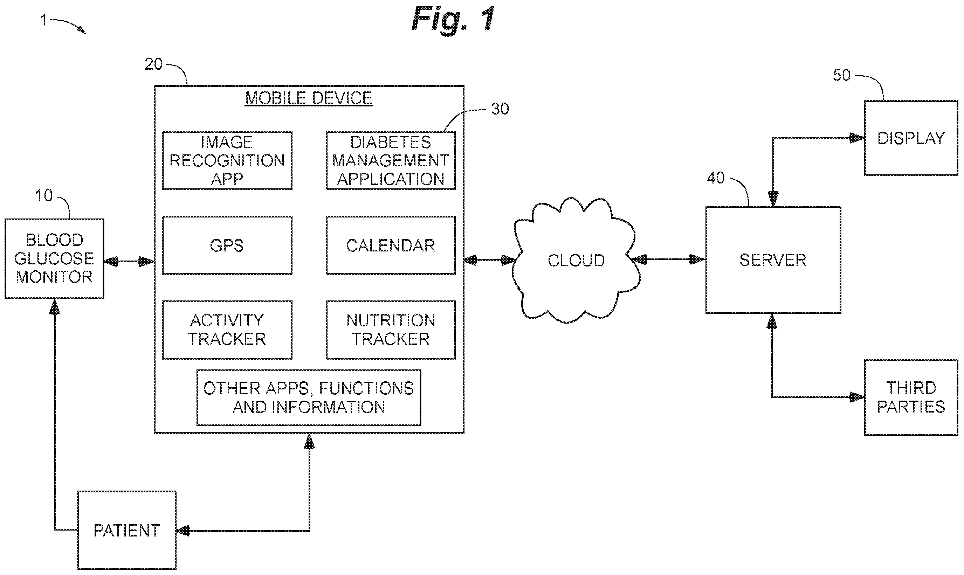

FIG. 1 is a schematic depiction of an ACC system 1. ACC system 1 includes a blood glucose monitor 10 used in conjunction with a mobile device 20. Mobile device 20 may include smartphones, tablets, computers, and other suitable mobile devices. In some embodiments, mobile device 20 may be a wearable device. Mobile devices can be convenient and advantageous for patients and other users because they can permit patients to access an ACC system 1 from a plurality of locations rather than from only a limited number of fixed locations.

Mobile device 20 may include various components or features. Components or features may include hardware, software, and combinations thereof. The scope of components and features on a mobile device will be recognized by those learned in the art. For example, mobile devices may include hardware and software to enable phone calls, SMS services, and cameras. In some embodiments, mobile device 20 includes diabetes management application 30. Mobile device 20 may include one or more of the following: a calendar application; an activity tracking application; a nutrition tracking application; or an image recognition application. Mobile device 20 may include system software, such as an operating system or firmware.

ACC system 1 may include a server 40 of ACC system 1. Server 40 may be remotely located from the mobile device 20. Server 40 may permit ACC system 1 to connect to a number of mobile devices 20 and to a number of provider devices located in remote locations. Server 40 may perform processes that are too complex for a mobile device 20 or require more data than mobile device 20 may reasonably store or access.

In some embodiments, ACC system 1 includes a display 50. Display 50 may comprise a tablet, smart phone, computer, or other device. Display 50 may comprise a browser or application capable of being viewed on a number of different devices. Server 40 may communicate with display 50 remotely.

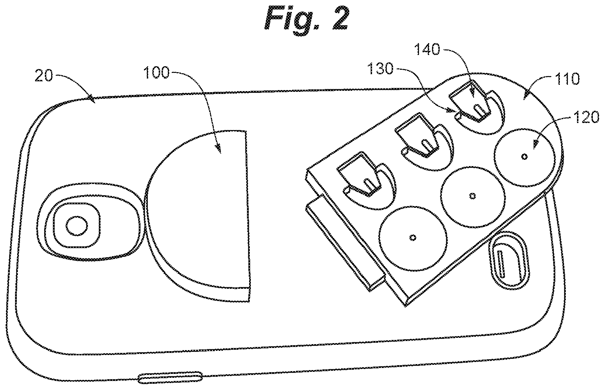

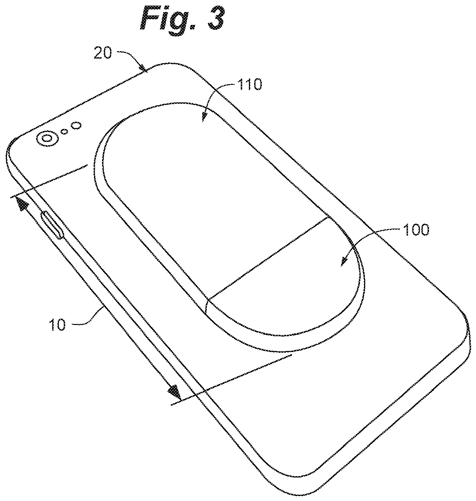

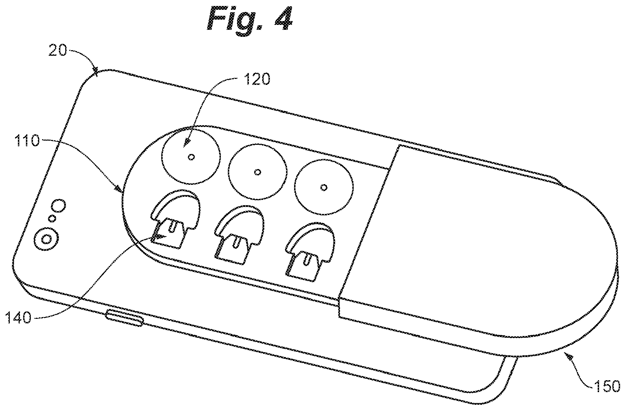

FIGS. 2-4 illustrate embodiments of blood glucose monitor 10. In many cases, blood glucose monitor 10 can be removably attached to mobile device 20. The blood glucose monitor 10 can include a reusable module 100, which may have measuring equipment configured to measure a property of a biological sample (e.g., a blood sample). The blood glucose monitor 10 can include a disposable module 110 with one or more lancet stations 120 and one or more test strip stations 130. Each lancet station 120 can include an enclosed lancet. Each test strip station 130 can include a biological strip 140. In some embodiments, the blood glucose monitor 10 includes a cover 150 (see Fig. 4) that can be removed to reveal the lancet stations 120 and the test strip stations 130. The disposable module 110 can be couplable to the reusable module 100. When the lancet stations 120 and test strip stations 130 of the disposable module 110 have been used, the disposable module 110 can be decoupled from the reusable module 100, and a new disposable module can be coupled to the reusable module 100. The blood glucose monitor is described in greater detail in U.S. Patent Application No. 13/946,838, which is titled "Blood Glucose Management" and co-owned with the present application, and which is hereby incorporated by reference herein in its entirety. U.S. Patent No. 8,647,357, which is titled "Lancet Device with Flexible Cover" and is likewise co-owned with the present application, also provides additional detail on the blood glucose monitor and is also hereby incorporated by reference herein in its entirety.

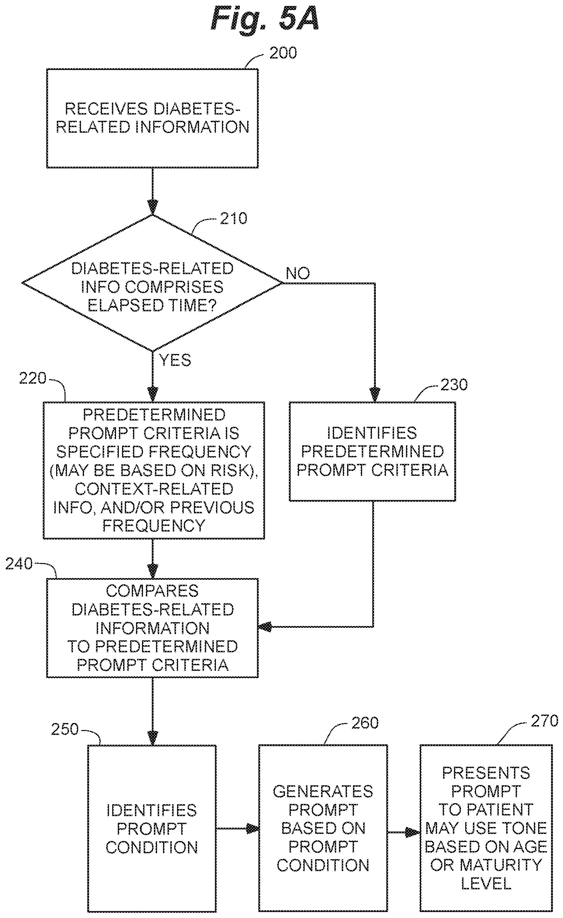

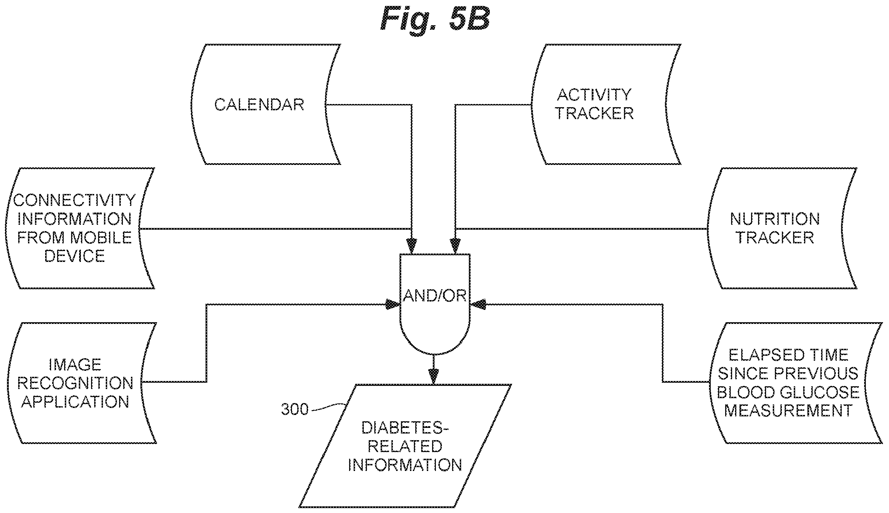

FIGS. 5A depicts a flowchart illustrating how a patient may be prompted to take a blood glucose management action. Diabetes-related information (300 of FIG. 5B) may be received from a mobile device 20 via diabetes management application 30. (200) Diabetes management application 30 may collect diabetes-related information 300 from a variety of sources, including by using components and functions of mobile device 20, and including combinations of various diabetes-related information discussed herein.

FIG. 5B is a flowchart depicting possible sources of diabetes-related information 300. The diabetes-related information 300 collected may include calendar information from a calendar application on mobile device 20. According to some embodiments calendar information may include an appointment scheduled near when a blood glucose measurement was received (e.g., a soccer practice within 30 minutes). Diabetes-related information 300 may include activity information from an activity tracking application on mobile device 20. In some embodiments activity information may include an activity level near when the blood glucose measurement was received (e.g., a pedometer application indicating that the patient just walked an abnormally long distance). Diabetes-related information 300 may include nutrition information from a nutrition tracking application on mobile device 20. Nutrition information may include food or drink consumed near when a blood glucose measurement was received. Diabetes-related information 300 may include image recognition information from an image recognition application on mobile device 20 (e.g., recognition of a particular fast food restaurant's logo in a recently taken photo). Diabetes-related information 300 may include mobile device connectivity information from mobile device 20. Mobile device connectivity information may include an indication that the mobile device has connected with another electronic device (e.g., a bicycle sensor, a vehicle electronics system, a router, etc.) via Wi-Fi or via short-range wireless communication protocol. Diabetes-related information 300 may include location information, such as GPS coordinates. Diabetes-related information 300 may include an elapsed time since diabetes management application 30 received a previous blood glucose measurement from a patient.

According to some embodiments, diabetes-related information 300 includes patient-provided answers to a context-related question subset. In some embodiments, the context-related information includes patient-provided answers to one or more context-related questions. In some of these embodiments, the patient-provided answers include an explanation of the previous frequency of blood glucose measurements of the patient. Further information relating to context-related questions are described further herein.

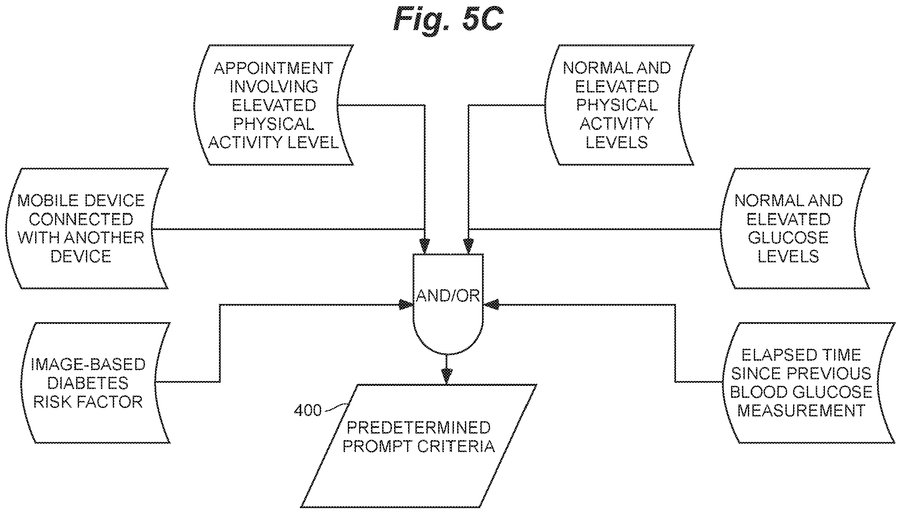

Diabetes management application 30 may include one or more predetermined prompt criteria (400 of FIG. 5C). Predetermined prompt criteria 400 may differ based on the type of diabetes-related information 300. For example, diabetes-related information 300 received may include calendar information from the calendar application on a mobile device. In these embodiments, the one or more predetermined prompt criteria 400 may include a calendar appointment type scheduled for a specified amount of time of a specific current time. In these embodiments, the one or more predetermined prompt criteria 400 may include a calendar appointment type comprising an appointment involving an elevated level of physical activity compared to a normal physical activity level for the patient.

FIG. 5C is a flowchart depicting other or additional types of predetermined prompt criteria 400. The diabetes-related information 300 received may include activity information from the activity tracking application on a mobile device 20. In these embodiments, the one or more predetermined prompt criteria 400 can include normal and elevated physical activity levels for the patient. The diabetes-related information 300 may include nutrition information from the nutrition tracking application on a mobile device 20. In some of these embodiments, the one or more predetermined prompt criteria 400 includes normal and elevated nutritional glucose levels of the patient. The diabetes-related information 300 may include image recognition information from the image recognition application on a mobile device 20. According to some of these embodiments, the one or more predetermined prompt criteria 400 includes an image-based diabetes risk factor. The diabetes-related information 300 may include mobile device connectivity information. In these embodiments, the one or more predetermined prompt criteria 400 can include an indication that the mobile device has connected with another electronic device.

Referring again to FIG. 5A, received diabetes-related information 300 may include the elapsed time since diabetes management application 30 received a previous blood glucose measurement from the patient. (210) In some of these embodiments, the one or more predetermined prompt criteria 400 comprises a specified frequency of blood glucose measurements. The specified frequency of blood glucose measurements may be tailored to the patient based on previous blood glucose measurements of the patient, previously collected context-related information, a previous frequency of blood glucose measurements of the patient, or combinations thereof. Whether or not diabetes-related information 300 includes a specified frequency, diabetes management application 30 may identify the appropriate predetermined prompt criteria 400.

Diabetes management application 30 may make a comparison of the received diabetes-related information 300 to one or more predetermined prompt criteria 400. (240) In some embodiments, diabetes management application 30 may identify a blood glucose management action prompt condition. (250) A prompt condition may be identified based on the comparison of diabetes-related information 300 to predetermined prompt criteria 400. For example, if diabetes-related information 300 includes calendar information and predetermined criteria includes a calendar appointment type, diabetes management application 30 may analyze the received calendar information to determine whether it includes calendar appointments of the specified type.

Diabetes management application 30 may generate a blood glucose management action prompt. (260) A prompt may be based on the blood glucose management action prompt condition. In some embodiments, diabetes management application 30 presents the blood glucose management action prompt to a patient via the user interface of a mobile device 20. (270) The blood glucose management action prompt may comprise a prompt to take a blood glucose measurement, a prompt to take in carbohydrates, a prompt to take in insulin, a prompt to exercise, or any combination thereof. Prompts may be presented using a prompt tone based on patient age or maturity level. Using different tones based on the patient may be utilized to increase patient engagement.

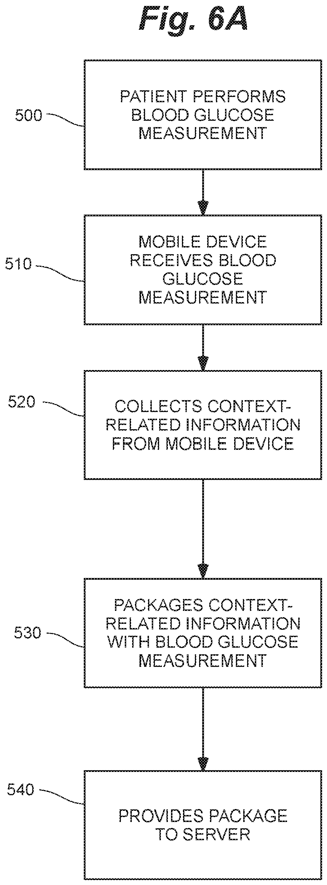

FIG. 6A is a flowchart depicting how information within ACC system 1 may be processed in some embodiments. Mobile device 20 may receive a blood glucose measurement from a patient. In some embodiments, a patient performs a blood glucose measurement using blood glucose monitor 10. (500) The blood glucose measurement may be received from blood glucose monitor 10 at diabetes management application 30 on mobile device 20. (510) The operation of the blood glucose monitor and its connections to the mobile device are detailed further herein.

In some embodiments, ACC system 1 can collect context-related information 700 concerning the blood glucose measurement. (520) The context-related information can provide context for the corresponding blood glucose measurement, which can allow for more informed diabetes management decisions and actions, along with better overall diabetes care. Context-related information 700 may be collected with the diabetes management application 30. Diabetes management application 30 may collect context-related information 700 from a variety of sources, including by using components and functions of mobile device 20.

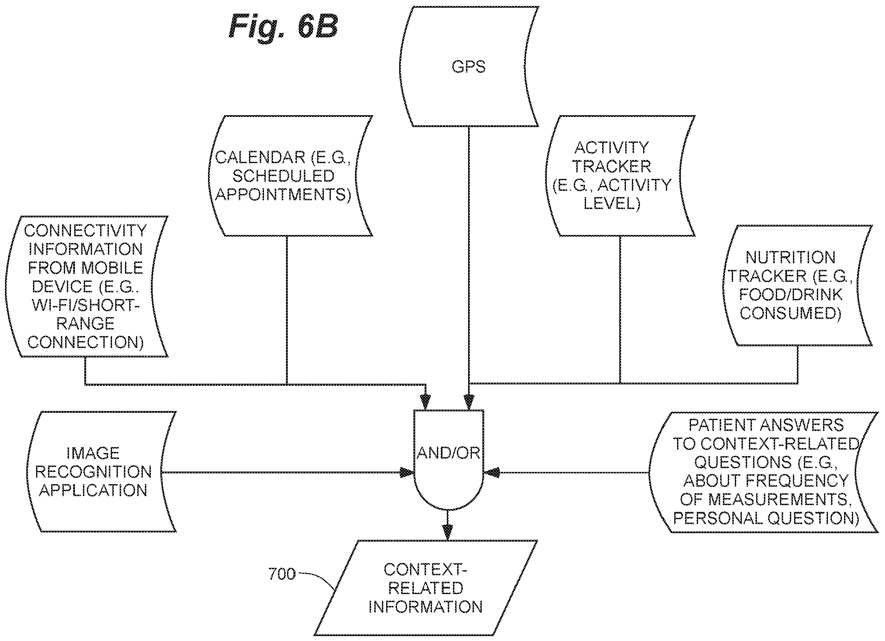

FIG. 6B is a flowchart depicting possible sources of context-related information 700. The context-related information 700 collected may include calendar information from a calendar application on mobile device 20. According to some embodiments calendar information may include an appointment scheduled near when a blood glucose measurement was received (e.g., a lunch scheduled at a particular restaurant). Context-related information 700 may include activity information from an activity tracking application on mobile device 20. In some embodiments activity information may include an activity level near when the blood glucose measurement was received. Context-related information 700 can also include nutrition information from a nutrition tracking application on mobile device 20. Nutrition information may include food or drink consumed near when a blood glucose measurement was received. Context-related information 700 may also include image recognition information from an image recognition application on mobile device 20. Context-related information 700 can include mobile device connectivity information from mobile device 20. Mobile device connectivity information may include an indication that the mobile device has connected with another electronic device via Wi-Fi or via short-range wireless communication protocol. Context-related information 700 may include location information, such as GPS coordinates.

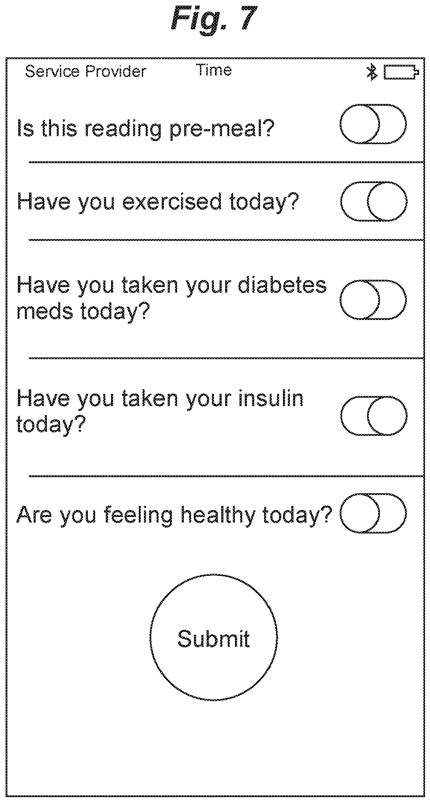

According to some embodiments, context-related information 700 includes patient-provided answers to a context-related question subset, or combinations thereof. FIG. 7 depicts how answers may be obtained from the context-related question subset in some embodiments.

ACC system 1 may select a context-related question subset from a context-related question set. (800) Selection may be performed by diabetes management application 30. In some embodiments, a context-related question subset is selected based on the received blood glucose measurement of the patient. In some embodiments, a context-related question subset is selected using a frequency of receiving blood glucose measurements of the patient. A context-related question subset may be selected based on a time of day of the received blood glucose measurement, or in some embodiments, based on previous answers to context-related questions and/or previous blood glucose measurements. (810)

Context-related questions may include health- and behavior-related questions. Context-related questions may include questions related to the diet, behavior, health, and/or symptoms of the patient. Context-related questions may include questions about the frequency of receiving blood glucose measurements of the patient and/or time of day of the received blood glucose measurement. Examples include:
- Have you checked your feet today?
- Do you have any sores on your feet?
- Have you had a professional check your feet?
- Have you had a professional check your eyes?
- Have you had diabetes education?
- Have you had dental exam?
- Is this reading pre-meal?
- Is this reading post-meal?
- Have you exercised today?
- Have you taken your diabetes medications today?
- Have you taken your medications today?
- Have you taken your insulin today?
- Did you take insulin within a time period before this reading?
- Are you feeling well today?
- Is your breathing normal?
- Do you have any numbness or tingling in your feet?
- Do you have any numbness or tingling in your fingers?
- Are your bowel movements normal?
- Have you had any low blood sugar moments?
- Have you lost consciousness or don't recall a period of time due to blood sugar?
- Are you struggling to keep your blood glucose in control?
- Are you struggling to count carbs?
- Are you struggling to calculate insulin amounts?

In some embodiments, the context-related question subset includes a personal question unrelated to health measurements. Questions may be presented using a question tone based on patient age or maturity level. Including questions unrelated to health measurements and using different tones based on the patient may be utilized to increase patient engagement.

Personal questions may relate to activities or items that the patient enjoys. For example, a question may be presented on whether a user has a pet or has a favorite sports team. Future questions or content may be added based on answers to personal questions. For example, future questions may inquire into a pets name if a patient indicates that he or she has a pet. Similarly, future questions may ask about recent games of a favorite sports team.

The tone of questions may be varied based on the patient's age or maturity level. For example, for young children simple sentence structures may be used and the tone of the questions can become friendlier or more enthusiastic. Conversely, for an older patient or patient who wishes to have a more "removed" experience, formal language and forms of address may be used.

Methods to increase patient engagement are important in the area of diabetes management. One of the most important factors in keeping a patient's diabetes under control is simply ensuring that they continue to monitor their health and blood glucose measurements. Customizing content and language to a patient's needs can assist in keeping patients engaged and interested in tracking their health measures.

ACC system 1 may present the context-related question subset to the patient. (820) The diabetes management application 30 may present the selected context-related question subset to the patient on a user interface of the mobile device 20. FIG. 7 is an example user interface of diabetes management application 30 posing context-related questions to the patient.

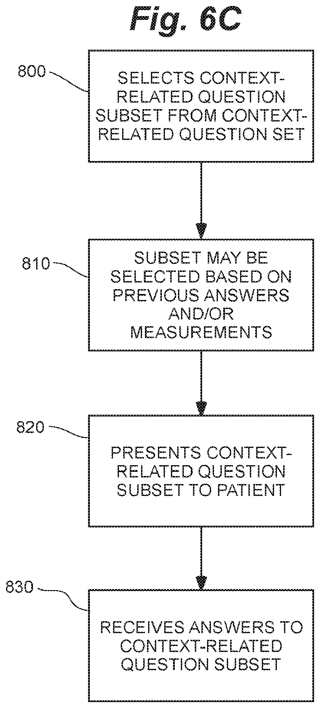

Referring again to FIG. 6C, the diabetes management application 30 may receive answers to the context-related question subset from the patient via the user interface. (830) In example user interfaces like that of FIG. 7, the patient can answer by sliding an on-screen bar to select yes or no. The question user interface depicted in FIG. 7 also depicts a "Submit" button that patients can use to finalize their answers for the diabetes management application 30.

Referring now to FIG. 6A, in some embodiments, collected context-related information 700 may be packaged with the received blood glucose measurement of the patient. (530) Diabetes management application 30 may perform the process of packaging the collected context-related information 700 with the blood glucose measurement of the patient.

The packaged context-related information 700 and blood glucose measurements of the patient may be provided to a server 40. (540) The diabetes management application 30 may provide the packaged context-related information 700 to server 40. Server 40 may be remotely located.

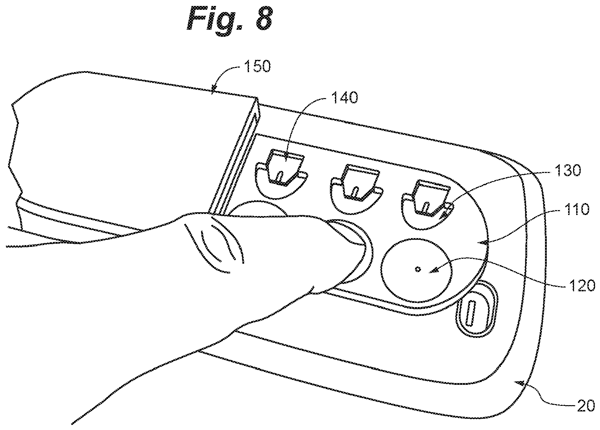

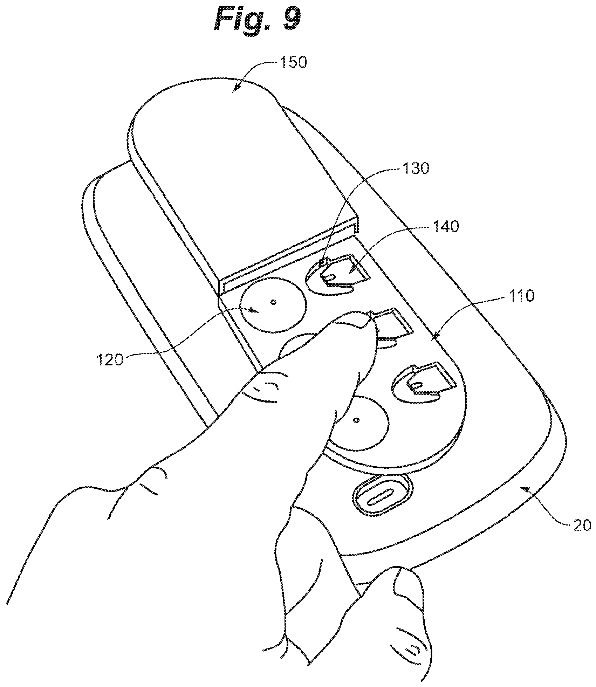

FIGS.8-9 show a patient using the blood glucose monitor 10. FIG. 8 shows the cover 150 removed and a patient pressing his/her finger on a lancet station 120. In doing so, the patient can lance his/her finger on a lancet enclosed in the lancet station 120. FIG. 9 shows the patient applying a blood sample generated from the lancing action to a biological strip 140. The biological strip 140 can be coupled to the measuring equipment of the reusable module 100, and the measuring equipment can determine a blood glucose measurement (or other biological sample measurement). In preferred embodiments, the reusable module 100 can transmit a signal representative of the blood glucose measurement to the mobile device 20 (or to a separate device) for display. The reusable module 100 can include a wireless transmitter (e.g., a Bluetooth transmitter) that can be configured to communicate with components in the mobile device 20 (or other device). Again, additional detail on use of the blood glucose monitor 10 can be found in the above-referenced U.S. Patent Application No. 13/946,838 and U.S. Patent No. 8,647,357.

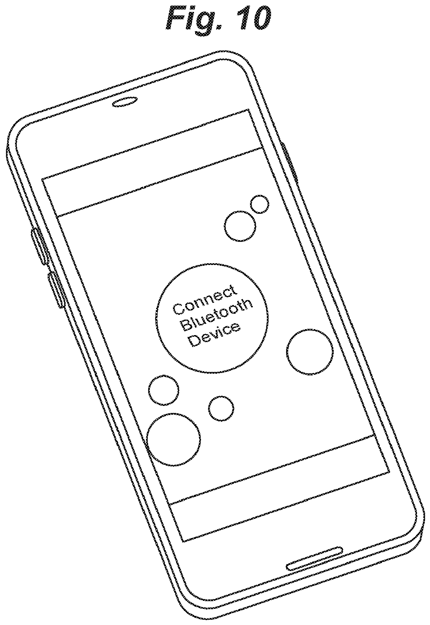

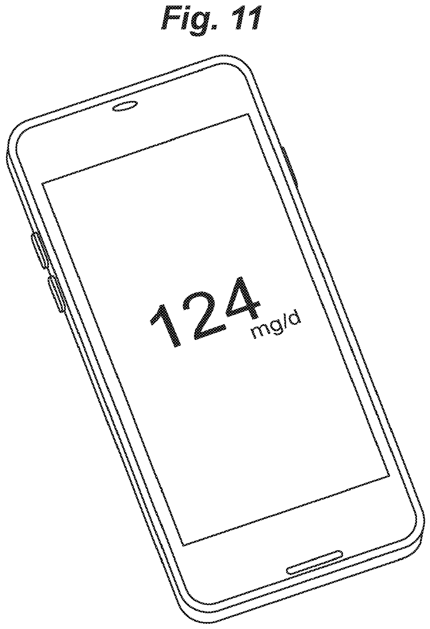

In some embodiments, mobile device 20 includes or utilizes a user interface. FIG. 10 is an example of a user interface from diabetes management application 30, a portion of the ACC system 1, requesting that the patient connect via Bluetooth to a device. In many embodiments, the device can be the lancet station discussed above in FIGS. 2-4 and 8-9. Referring again to FIG. 10, the patient can press the "Connect Bluetooth Device" button to activate the connection. When the patient activates this connection, a signal representative of the blood glucose measurement can be transmitted to the mobile device. Diabetes management application 30 can then run an algorithm to analyze the signal. FIG. 11 is an example user interface of diabetes management application 30 displaying the blood glucose measurement.

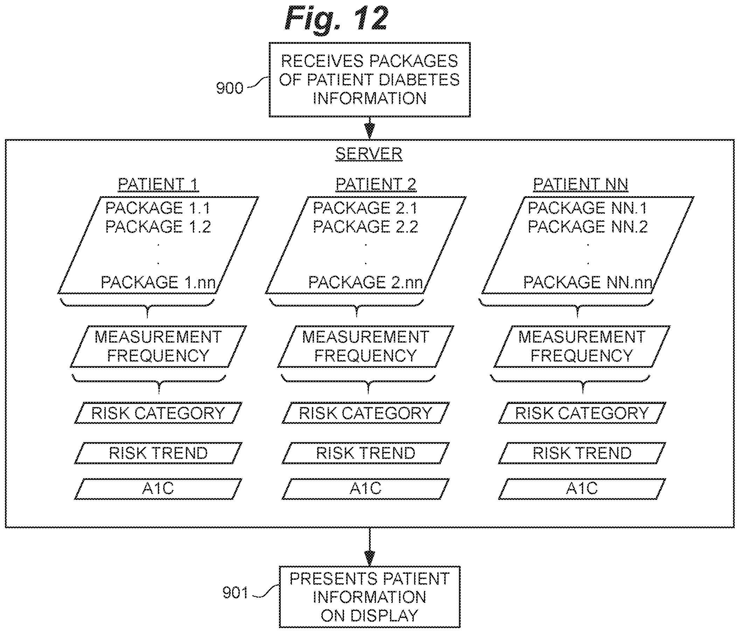

FIG. 12 is an illustration of how information is processed using a server in some embodiments. Server 40 may receive information from diabetes management application 30. In some embodiments, server 40 may receive packages of patient diabetes information from a plurality of patients. (900) In these embodiments, each package of patient diabetes information may be provided by a diabetes management application 30 on a mobile device 20 of a patient.

In many embodiments, each package of patient diabetes information comprises a blood glucose measurement and context-related information 700 of a patient. As is depicted in FIG. 12, server 40 may store packages of patient diabetes information from a number of patients, for example from a patient number 1 to a patient number NN. The server may store multiple packages of patient diabetes information for each patient, for example from package number 1 to package number nn. Patients may have differing amounts and types of information. For example, patient 2 in FIG. 12 is depicted as having sent only two packages. It will be understood by practitioners learned in the art that packages of data may be stored in a number of manners, including by utilizing a database or other storage system. Patient diabetes information may be processed or stored in ways other than or in addition to using packages or in order of package receipt.

In many embodiments, the diabetes management application 30 can send additional information with the context-related information 700 and the blood glucose measurement, such as the time of day, activity level, GPS location, weather, home automation data, shopping lists and other information created by internet connected personal assistants such as Amazon Echo, and other information. In some situations, such additional information can constitute context-related information. This transmission can be via Wi-Fi, telephone connection, or other suitable communication channel. The transmission can be in an encrypted form. The ACC server can verify the transmitted information according to the patient's serial number, index the information to the patient, and store the information according to the patient's clinic on server databases. In some systems, the diabetes management application 30 can gather the blood glucose measurement, the context-related information 700, and the above-referenced additional information from the patient and can function independently of any ACC server to help the patient manage his/her blood glucose levels.

In ACC system 1, both the diabetes management application 30 and server 40 can be configured to perform a variety of analyses. The server 40 may compare the packages of patient diabetes information with one or more previously received packages of patient diabetes information. The server 40 may determine a blood glucose measurement frequency for each patient using the comparison.

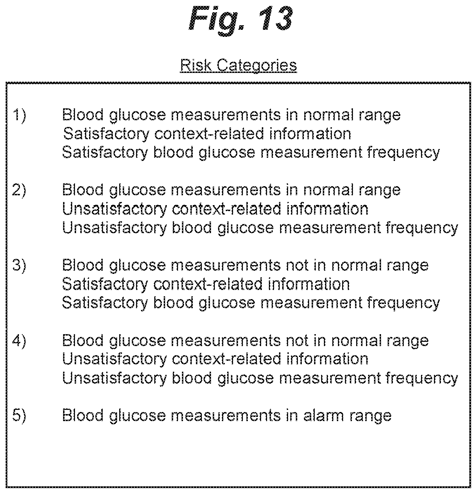

The server 40 may categorize the plurality of patients into various diabetes risk categories. The categorization of each patient may be based on the patient's package of patient diabetes information, the patient's one or more previously received packages of patient diabetes information, and the patient's blood glucose measurement frequency. The categories may comprise five diabetes risk categories. FIG. 13 includes a chart describing an embodiment of risk categories. Risk categories may include, from lowest risk to highest risk:
(i) blood glucose measurements in normal range and satisfactory answers to context-related information and blood glucose measurement frequency,
(ii) blood glucose measurements in normal range and unsatisfactory context-related information and blood glucose measurement frequency,
(iii) blood glucose measurements not in normal range and satisfactory context-related information and blood glucose measurement frequency,
(iv) blood glucose measurements not in normal range and unsatisfactory context-related information and blood glucose measurement frequency, and
(v) blood glucose measurement in alarm range.

Returning to FIG. 12, in some embodiments, server 40 may identify a trend of a patient moving from one diabetes risk category to another. In some cases is may also identify a trend of a patient becoming more or less at risk within a diabetes risk category.

Server 40 analysis may include projecting an A1C level for at least one patient and presenting the projected A1C level on the display. The A1C level is a measurement used to identify a patient's three month average plasma glucose concentration.

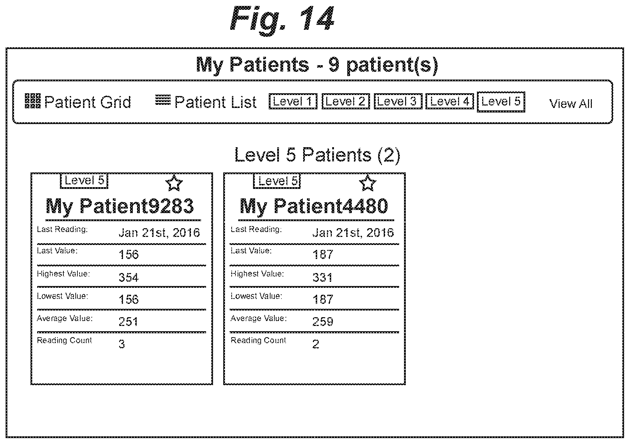

Patient information for at least some of the patients may be presented on a display of the ACC system 1 with the server 40. (910) Patient information displayed may include identity information for the patient, blood glucose measurement information for the patient, and the patient's diabetes risk category. FIG. 14 illustrates and example display. Patient information may be displayed in a grid configuration. Patients may be displayed in a list configuration. Users may toggle between types of displays by selecting one of the display buttons on the top of the screen. The display of FIG. 14 allows a user to filter the patients shown by one or more risk categories by selecting one of a number of buttons along the top of the screen. The user may view all patients by selecting a "View All" button.

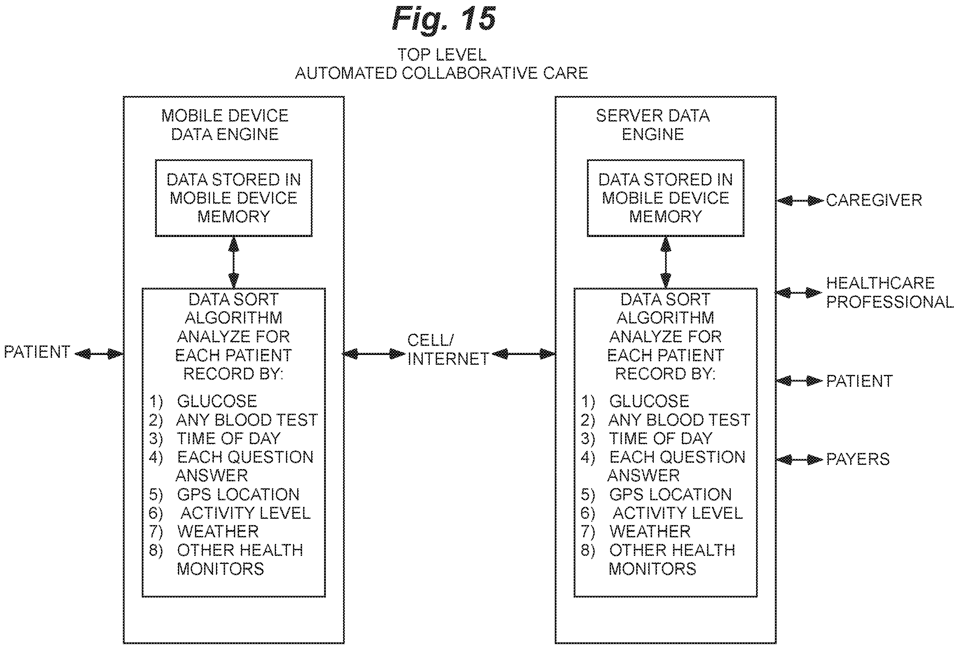

As FIG. 15 illustrates, both the diabetes management application 30 and server 40 can analyze for each patient record by (1) glucose, (2) any blood test, (3) time of day, (4), each question answer, (5) GPS location, (6) activity level, (7) weather, and/or (8) other health monitors. A variety of users can access relevant information via ACC system 1, including patient-approved caregivers, healthcare professionals, patients, market researchers, advertisers, business providing application, other businesses, governments, and payers.

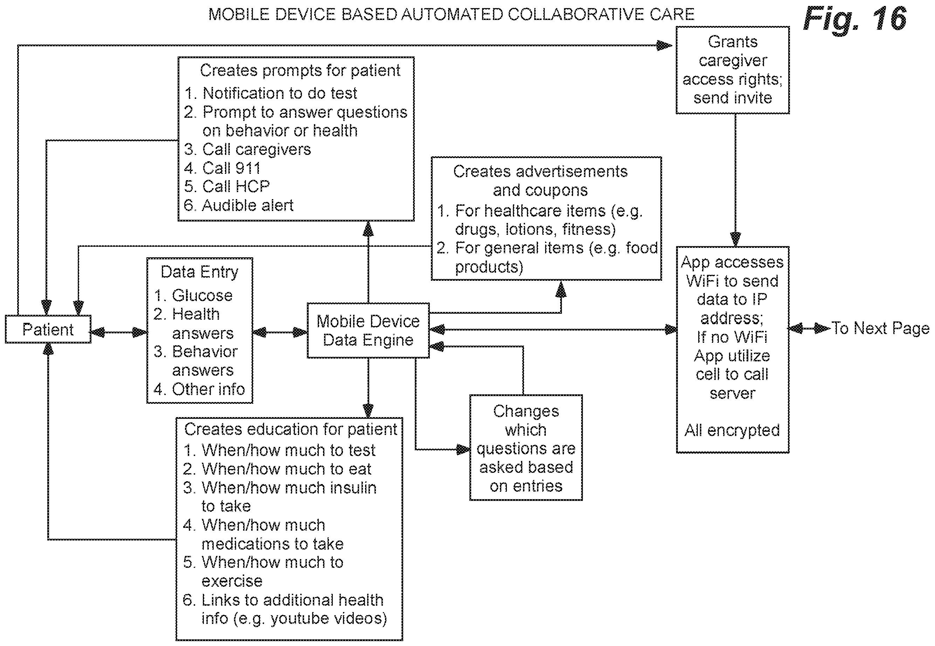

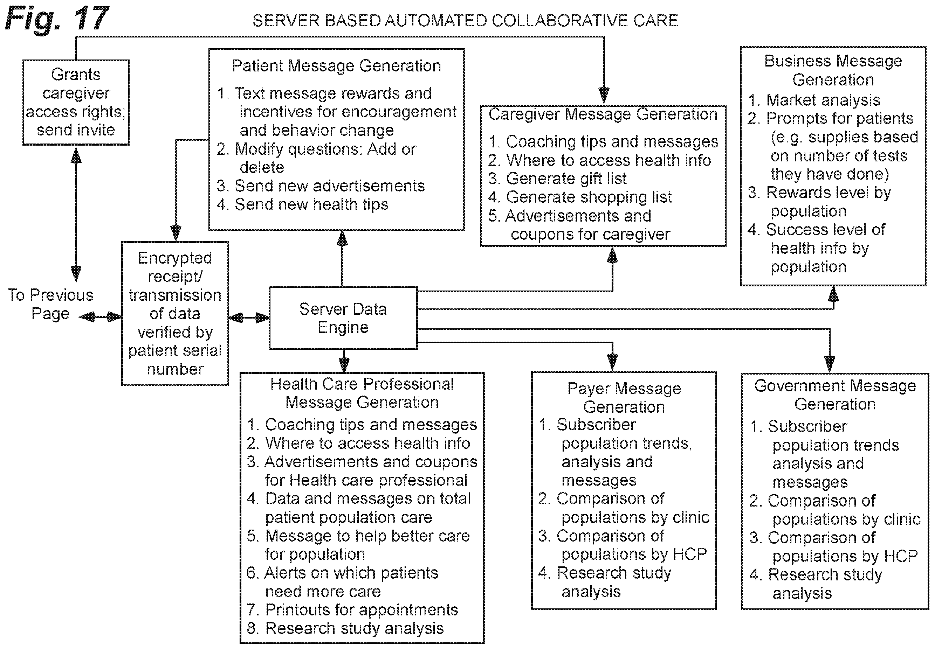

Together, FIGS. 16-17 illustrate operation of an ACC system 1. FIG. 10 shows operation of a diabetes management application 30. FIG. 17 shows operation of an embodiment of a server.

Referring to FIG. 17, the patient can interact with a data entry user interface, and diabetes management application 30 can take a variety of actions based on input provided by the patient. As shown, the diabetes management application 30 can determine whether to change which context-related questions are presented to the patient. This determination can be based on entries previously provided by the patient. As shown, the diabetes management application 30 can generate prompts for the patient. Examples of prompts include (a) a notification to take a blood glucose measurement (e.g., based on a preset or calculated time), (b) a prompt to answer various behavior- and health-related questions (e.g., upon transmission of a blood glucose measurement, according to a preset or calculated time, or based on other factors), (c) a recommendation to alert other parties (e.g., call a caregiver, call a healthcare provider, call 911). In many embodiments, such prompts can come in the form of a displayed message to the patient. In some embodiments, such prompts can include an audible alert.

The diabetes management application 30 shown in FIG. 16 can display messages, text, images, and/or other content to the patient based on input provided by the patient. Examples of displays to patients may include educational information, advertisements, alerts, and rewards. Displays may be personalized based on received information. Examples of information from which displays may be personalized include the collected context-related information 700 or previously collected context-related information 700. Displays may also be personalized based on current received blood glucose measurements and previous blood glucose measurements of the patient. Displays may also be based on any combination of this information.

One type of display is educational information for the patient on various topics. Examples of such educational information include when or how often to take blood glucose measurements, when or how often to eat or exercise, when or how much insulin or medications to take, and links to additional online health information.

A type of display may include tailored advertisements and coupons related both to specific healthcare items (e.g., drugs, lotions, fitness items, etc.) and to general items (e.g., food products). Advertisements may be based on information obtained from blood glucose monitor 10, such as information that blood glucose monitor 10 has a low number of items used for testing blood glucose levels.

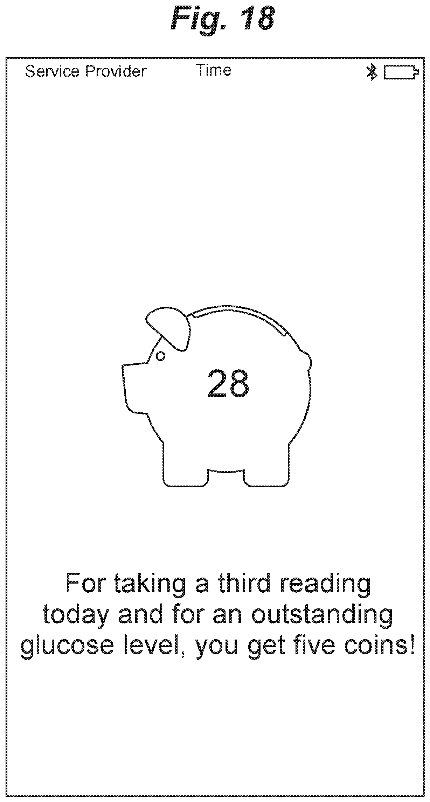

One type of display is rewards. FIG. 18 is an example user interface of diabetes management application 30 displaying in-application rewards granted to patient based on positive patient behaviors. The server 40 can include functions to handle a rewards program. Upon patients submitting data to server 40, server 40 can determine whether certain conditions have been met (e.g., submitting timely measurements, engaging in positive behavior, achieving positive measurements, giving positive answers to the context-specific questions posed by the diabetes management application 30, etc.). Upon a condition being met, server 40 can add set levels of points to the patient's rewards account. The server 40 can store the rewards points information for each patient and additionally send information to the diabetes management application 30 indicating points and rewards achieved. Upon achieving a certain number of points, patients can be eligible for certain rewards, or may trade the points in for rewards. The rewards can include coins or prize money tied to the diabetes management application 30.

As shown, the diabetes management application 30 can allow the patient to manage who is able to access his/her account. The diabetes management application 30 can enable the patient to input information on caregivers and grant those caregivers access rights to the patient's information accessible via ACC caregiver software. Upon the patient granting new access rights, the diabetes management application 30 can send the entered information to the server 40, which can store the information. The diabetes management application 30 can offer the patient the option to send invitations to potential caregiver-users. Upon the patient selecting the option to send an invitation, the diabetes management application 30 can send an email or other message to the caregiver with information on how to download the ACC caregiver software.

Referring to FIGS. 16-17, the diabetes management application 30 of FIG. 16 can communicate with server 40. Communication can be via Wi-Fi and/or cellular data. In many embodiments, communication can be encrypted. Upon receipt of encrypted communication, server 40 can verify the communication by the patient's serial number.

As is discussed further herein, server 40 may be able to provide information to a variety of users. In some instances, the server 40 can provide information to a patient only if a predetermined condition is met-e.g., if a blood glucose measurement is above a certain threshold. In some instances, the ACC server can provide information to a patient whenever analysis is completed-e.g., full blood glucose measurement information can be provided to the diabetes management application 30 or software of a patient's healthcare provider after each analysis.

As shown, server 40 can provide information to the patient. The patient can receive information when a number of different conditions are met. If server 40 determines that reward conditions (discussed in greater detail elsewhere herein) are met, server 40 can send a message to the patient's diabetes management application 30 or send a text message to the patient's phone number stating that the patient has met a goal or achieved a reward. In some instances, server 40 can send messages to the patient's diabetes management application 30 or send a text message offering incentives for encouraging behavior changes. In some instances, server 40 can send content for the diabetes management application 30 to display advertisements, coupons, and educational materials, as discussed elsewhere herein. In some instances, server 40 can be configured to add or delete questions from those stored on the diabetes management application 30, as discussed elsewhere herein.

Server 40 can provide information to healthcare professionals. Such healthcare professionals can communicate with server 40 via ACC healthcare professionals software. The server 40 can send healthcare professionals clinical patient data, including test results, time of day, and other information. The server 40 can send healthcare professionals a message when a patient has received a health alert from the ACC system 1. The server 40 can analyze the data collected from all of a healthcare professional's patients who use ACC system 1 and send information based on the data analysis to healthcare professional. Such information can include recommendations for how healthcare professionals can improve the care available to their overall patient population. The ACC healthcare professionals software can include features to make patient data sortable and presentable according to any of a variety of categories of data (e.g., day of test, answer to specific questions, time of test, etc.).

The server 40 can send healthcare professionals various messages. Such messages can include coaching tips and explanations of individual patient data and blood glucose measurements for the healthcare professional to convey to the patient's diabetes management application 30. Such messages can include other information for healthcare professionals to convey to patients. In some instances, server 40 can provide a healthcare professional tailored advertisements and coupons. The server 40 can inform healthcare professionals of trends related to total patient population care and related tips on caring for the healthcare professional's overall patient population. In some instances, server 40 can provide alerts on which patients need additional care, along with printouts to aid in appointments and research study analysis.

As shown, server 40 can provide information to caregivers (e.g., through ACC caregivers software). Such information can include coaching tips and messages for the caregiver to relay to the patient. In some instances, the caregiver can relay messages to the patient via direct messages that are viewable in the patient's diabetes management application 30.

The server 40 can provide information to payers (e.g., via ACC payers software). The server 40 can be configured to analyze overall patient data to generate subscriber population trends and analysis and can send such information to the payer. In some instances, server 40 can compute comparisons of patient populations by clinic or by healthcare provider and can send such comparison information to the payer. In some instances, server 40 can send messages to the payer related to research study analysis.

As shown, server 40 can provide information to various business entities. For example, an ACC administrator or other business can access information from the ACC server to conduct market analysis. An ACC administrator or other business can generate and deliver prompts to patients (e.g., supplies based on tests completed by the patients). In some instances, an ACC administrator or other business may be interested in learning the level of reward and/or the health success level of patients by population. In some instances, the ACC server can provide information.

The server 40 can provide information to government entities. The server 40 can be configured to analyze overall patient data to generate subscriber population trends and analysis and can send such information to a government entity. In some embodiments, server 40 can be configured to compute comparisons of patient populations by clinic or by healthcare provider and can send such comparison information to a government entity. In some instances, server 40 can send messages to the payer related to research study analysis.

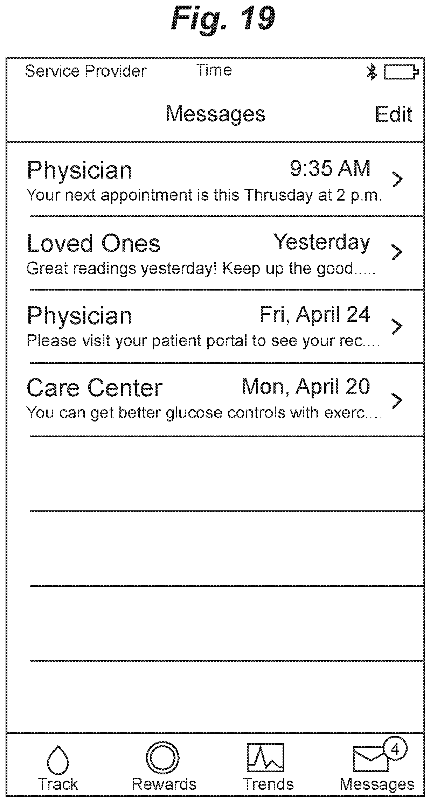

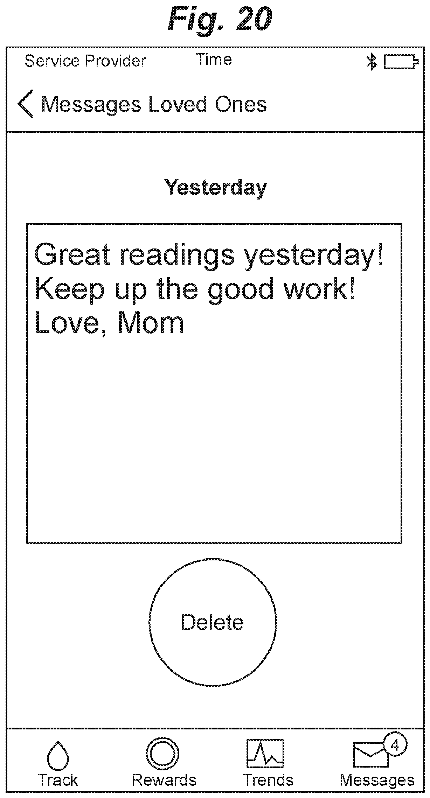

The diabetes management application 30 and ACC software can allow in-app/in-software messaging between the patient and other users. FIG. 19 is an example user interface of the diabetes management application 30 displaying messages a patient has received from several categories of persons or server 40. The server 40 can enable the patient's selected healthcare providers, caregivers, and others (e.g., loved ones) to send messages directly to the patient via the diabetes management application 30 (and vice versa). FIG. 20 is an example user interface of the diabetes management application 30 displaying an individual message, in this case from the category "Loved Ones."

In the foregoing detailed description, the invention has been described with reference to specific embodiments. However, it may be appreciated that various modifications and changes can be made without departing from the scope of the invention as set forth in the appended claims. Thus, some of the features of preferred embodiments described herein are not necessarily included in preferred embodiments of the invention which are intended for alternative uses.

## Claims

1. A method of diabetes management comprising:
receiving a blood glucose measurement of a patient from a blood glucose monitor at a diabetes management application on a mobile device;
collecting context-related information concerning the blood glucose measurement with the diabetes management application, the context-related information including patient-provided answer(s) to a context-related question subset, wherein the context-related question subset is selected by the diabetes management application from a context-related question set based on the received blood glucose measurement and a frequency of receiving blood glucose measurements of the patient;
packaging the collected context-related information with the received blood glucose measurement of the patient with the diabetes management application; and
providing the packaged context-related information and blood glucose measurement of the patient to a server of a remotely located automated collaborative care system with the diabetes management application.

2. The method of claim 1, wherein the context-related information includes the patient-provided answer(s) to the context-related question subset, the diabetes management application presenting the selected context-related question subset to the patient on a user interface of the mobile device and receiving answer(s) to the context-related question subset from the patient via the user interface.

3. The method of claim 2, wherein the context-related question subset is selected based additionally on previous answers by the patient to previously presented context-related questions and/or previous blood glucose measurements of the patient.

4. The method of claim 2, wherein the context-related question subset includes a question about the frequency of receiving blood glucose measurements of the patient.

5. The method of claim 2, wherein the context-related question subset includes a personal question unrelated to diabetes to increase patient engagement.

6. The method of claim 2, further comprising receiving an update to the context-related question set from the server at the diabetes management application.

7. The method of claim 2, wherein presenting the context-related question subset to the patient comprises using a question tone based on patient age or maturity level.

8. The method of claim 1, further comprising presenting an advertisement to the patient on the user interface with the diabetes management application, the advertisement being personalized to the patient based on the received blood glucose measurement of the patient, the collected context-related information, previous blood glucose measurements of the patient, previously collected context-related information, or combinations thereof.

9. The method of claim 1, further comprising presenting educational information to the patient on the user interface with the diabetes management application, the educational information being personalized to the patient based on the received blood glucose measurement of the patient, the collected context-related information, previous blood glucose measurements of the patient, previously collected context-related information, or combinations thereof.

10. The method of claim 1, further comprising presenting a reward to the patient on the user interface with the mobile device diabetes management application, the reward being personalized to the patient based on the received blood glucose measurement of the patient, the collected context-related information, previous blood glucose measurements of the patient, previously collected context-related information, or combinations thereof.

11. The method of claim 1, further comprising presenting an alert to the patient on the user interface with the diabetes management application, the alert being personalized to the patient based on the received blood glucose measurement of the patient, the collected context-related information, previous blood glucose measurements of the patient, previously collected context-related information, or combinations thereof.

12. The method of claim 1, further comprising prompting the patient to take a blood glucose measurement with the diabetes management application based on the received blood glucose measurement of the patient, the collected context-related information, previous blood glucose measurements of the patient, previously collected context-related information, or combinations thereof.

13. The method of claim 1, further comprising receiving an access rights grant from the patient at the diabetes management application via the user interface.

14. The method of claim 1, wherein the context-related information further includes calendar information from a calendar application on the mobile device, activity information from an activity tracking application on the mobile device, nutrition information from a nutrition tracking application on the mobile device, image recognition information from an image recognition application on the mobile device, mobile device connectivity information from the mobile device, or combinations thereof,
wherein the calendar information includes an appointment scheduled near when the blood glucose measurement was received,
wherein the activity information includes an activity level near when the blood glucose measurement was received, and
wherein the nutrition information includes food/drink consumed near when the blood glucose measurement was received.

15. The method of claim 1, wherein the mobile device connectivity information includes an indication that the mobile device has connected with another electronic device via short-range wireless communication protocol.
